# EUROPEAN PATENT APPLICATION

(11) **EP 1 142 561 A1**
(43) Date of publication of application: **10.10.2001**
(21) Application number: 99959947.5
(22) Date of filing: 21.12.1999
(51) Int. Cl.: A61K 7/13

(54) **HAIR DYE COMPOSITIONS**

(30) Priority: 22.12.1998 JP 36420198
(71) Applicant: Lion Corporation, Tokyo 130-8644 (JP)
(72) Inventor: ONUKI, Takeshi, Sumida-ku, Tokyo 130-8644 (JP); NOGUCHI, Mutsumi, Sumida-ku, Tokyo 130-8644 (JP); MITAMURA, Joji, Sumida-ku, Tokyo 130-8644 (JP)
(74) Representative: Stuart, Ian Alexander
(86) International application number: JP9907187
(87) International publication number: WO0037030

(57) **Abstract**

A laccase-containing oxidation hair dye composition of one-pack type in which carbonated water is used as a solvent.

## Description

### TECHNICAL FIELD

The present invention relates to an oxidation hair dye composition of one-pack type which is incorporated with laccase. More particularly, the present invention relates to an oxidation hair dye composition of one-pack type which keeps laccase stable for a long time and produces a good hair-dyeing effect.

### BACKGROUND ART

An ordinary oxidation hair dye is composed of a dye precursor (primary ingredient) and an oxidizing agent (secondary ingredient) which are mixed together for reaction before use and the resulting mixture is applied to white hair to be dyed.

The dye precursor as the primary ingredient is p-phenylenediamine, p-aminophenol, or the like. It is usually alkaline and hence it poses a problem with irritant action on the scalp.

The oxidizing agent as the secondary ingredient is usually hydrogen peroxide. It is known that hydrogen peroxide damages hair and damaged hair turns reddish-brown after continued use for a long time.

An attempt has been made to tackle the first problem by making the primary ingredient neutral (in Japanese Patent Laid-open No. Hei 8-217652). Another attempt has been made to tackle the second problem by replacing hydrogen peroxide with an oxidase. Examples of oxidases include peroxidase (Japanese Patent Laid-open Nos. Sho 47-10400 and Sho 53-32132), laccase (US Patent No. 3251742 and Japanese Patent Laid-open No. Hei 6-172145), and uricase (Japanese Patent Laid-open No. Sho 63-246313). These disclosed technologies still have disadvantages. That is, peroxidase needs hydrogen peroxide to be added to the hair dye system because of the inherent characteristics of enzyme. Also, uricase utilizes hydrogen peroxide evolved by the enzymatic reaction; therefore, it does not eliminate troubles due to hydrogen peroxide.

By contrast, laccase damages hair less than peroxidase and uricase because it does not need the hair dye system to contain hydrogen peroxide. The disadvantage of laccase used as an ingredient of a hair dye composition of one-pack type is that it reacts with the dye precursor in the composition, giving rise to insoluble aggregates, during storage, because it is an unstable protein, so that laccase cannot fully produce its effect. This is a serious problem to be solved before laccase is adopted for commercial hair dye compositions of one-pack type.

There have been disclosed technologies to improve the storage stability of oxidase such as catalase and uricase in Japanese Patent Laid-open Nos. Hei 8-175935 and Hei 8-217652, respectively. However, there is no known technology to improve the stability of laccase.

### DISCLOSURE OF INVENTION

It is an object of the present invention to provide a laccase-containing oxidation hair dye composition of one-pack type which keeps laccase stable, without forming aggregates, and permits laccase to fully produce its effect during manufacturing or storing the composition.

In order to achieve the above-mentioned object, the present inventors carried out extensive studies, which led to the finding that a laccase-containing hair dye composition of one-pack type keeps laccase very stable for a long time, significantly preventing the dye precursor from forming aggregates, and fully produces its hair dyeing effect if carbonated water is used as a solvent to dissolve the laccase and the dye precursor. The present invention is based on this finding.

It is an object of the present invention to provide a laccase-containing oxidation hair dye composition of one-pack type in which carbonated water is used as a solvent.

### BEST MODE FOR CARRYING OUT THE INVENTION

A detailed description of the invention is given below. According to the present invention, the hair dye composition contains laccase as an oxidizing agent. The laccase used in the present invention is an enzyme designated as E.C.1.10.3.2. Laccase oxidizes urushiol in the sap of lacquer tree, thereby forming Japanese lacquer. Laccase is also ubiquitous in many plants and microorganisms, and it catalyzes the oxidation of aromatic compounds. The origin of laccase is not restricted in the present invention.

The hair dye composition of the present invention may be incorporated with laccase in an amount of 0.0005 to 30% (by weight), preferably 0.005 to 20%, depending on the form of composition, the frequency of use, the time required for processing, and the enzyme potency. With an amount less than 0.0005%, laccase does not fully produce its effect. With an amount more than 30%, laccase does not produce any additional effect in proportion to the amount.

The hair dye composition of the present invention contains an dye precursor which is involved in color reaction with laccase. The dye precursor in the present invention is not specifically restricted in its kind and amount. It may be chosen from any known ones. It includes the following examples, which are listed in the standards of raw materials for quasi drugs: 5-amino-o-cresol, o-aminophenol, m-aminophenol, p-aminophenol, 2,6-diaminopyridine, 5-(2-hydroxyethylamino)-2-methylphenol, N,N-bis(β-hydroxyl)-p-phenylenediamine sulfate, p-nitro-o-phenylenediamine, p-phenylenediamine, m-phenylenediamine, N-phenyl-p-phenylenediamine, resorcinol, 2-hydroxy-5-nitro-2',4'-diaminobenzene sodium sulfate, toluene-2,5-diamine, 5-amino-o-cresol sulfate, p-aminophenol sulfate, o-chloro-p-phenylenediamine sulfate, 4,4'-diaminodiphenylamine sulfate, p-methylaminophenol sulfate, p-phenylenediamine sulfate, m-phenylenediamine sulfate, toluene-2,5-diamine sulfate, 2,4-diaminophenoxyethanol hydrochloride, toluene-2,5-diamine hydrochloride, m-phenylenediamine hydrochloride, 2,4-diaminophenol hydrochloride, 3,3'-iminodiphenol, p-phenylenediamine hydrochloride, N-phenyl-p-phenylenediamine hydrochloride, N-phenyl-p-phenylenediamine acetate, 1,5-dihydroxynaphthalene, toluene-3,4-diamine, p-methylaminophenol, N,N'-bis(4-aminophenyl)-2,5-diamino-1,4-quinonediimine, o-aminophenol sulfate, 2,4-diaminophenol sulfate, and m-aminophenol sulfate. They may be used in an adequate amount alone or in combination with one another.

The above-mentioned dye precursor may be used in combination with any of the following direct materials: 2-amino-4-nitrophenol, 2-amino-5-nitrophenol, 1-amino-4-methylaminoanthraquinone, nitro-p-phenylenediamine hydrochloride, 1,4-diaminoanthraquinone, nitro-p-phenylenediamine, picramic acid, sodium picramate, 2-amino-5-nitrophenol sulfate, resorcinol, nitro-p-phenylenediamine sulfate, p-nitro-o-phenylenediamine sulfate, and p-nitro-m-phenylenediamine sulfate.

Of the above-mentioned dye precursors, the following are particularly desirable: p-phenylenediamine or a salt thereof, toluene-2,5-diamine or a salt thereof, p-aminophenol, 5-amino-o-cresol, m-aminophenol, p-nitro-o-phenylenediamine, 2,6-diaminopyridine, resorcinol, o-aminophenol, and m-phenylenediamine.

A substance like a melanine precursor represented by the general formula (1) below is also used to meet the recent nature-oriented taste.

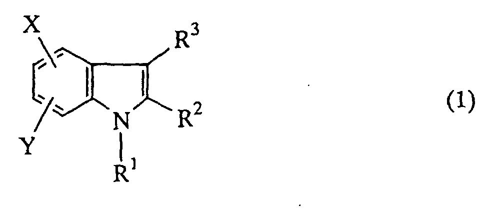

In the general formula (1), X denotes a hydrogen atom, NH₂, OH, or any of linear or branched alkyl group having 1 to 6 carbon atoms, alkenyl group, and alkoxyl group; and Y denotes OH or NH₂. If X is OH or any of linear or branched alkyl group having 1 to 6 carbon atoms, alkenyl group, and alkoxyl group, X is at the 5-, 6-, or 7-position of the ring and the ortho position with respect to Y.

R¹ and R³ are identical or different, each denoting a hydrogen atom or any of linear or branched alkyl group having 1 to 6 carbon atoms, alkenyl group, and alkoxyl group; and R² denotes any of linear or branched alkyl group having 1 to 6 carbon atoms, alkenyl group, alkoxyl group, and carboxyl group.

The compound represented by the general formula (1) above includes the following: 4,5-dihydroxyindole, 5,6-dihydroxyindole, 6,7-dihydroxyindole, N-methyl-5,6-dihydroxyindole, N-ethyl-5,6-dihydroxylindole, N-hexyl-5,6-dihydroxyindole, 2-methyl-5,6-dihydroxyindole, 3-methyl-5,6-dihydrxoxyindole, 4-hydroxyindole, 2,3-dimethyl-5,6-dihydroxyindole, 2-methyl-5-ethyl-6-hydroxyindole, 2-methyl-5-hydroxy-6-β-hydroxyethylindole, 4-hydroxypropylindole, 2-hydroxy-3-methoxyindole, 4-hydroxy-5-methoxyindole, 6-hydroxy-7-methoxyindole, 6-hydroxy-5-methoxyindole, 6-hydroxyindole, 5-hydroxyindole, 7-hydroxyindole, 7-aminoindole, 5-aminoindole, 4-aminoindole, 5,6-dihydroxyindole carboxylic acid, and 1-methyl-5,6-dihydroxyindole and a salt thereof.

A substance like a melanine precursor represented by the general formula (2) below is also desirable.

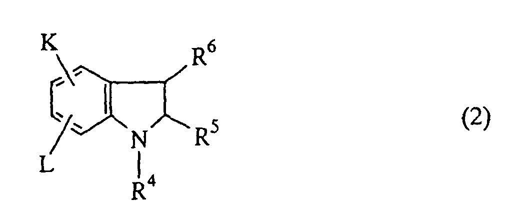

In the general formula (2), K denotes a hydrogen atom, NH₂, OH, or any of linear or branched alkyl group having 1 to 6 carbon atoms, alkenyl group, and alkoxyl group; and L denotes OH or NH₂. If K is OH or any of linear or branched alkyl group having 1 to 6 carbon atoms, alkenyl group, and alkoxyl group, K is at the 5-, 6-, or 7-position of the ring and the ortho position with respect to L.

R⁴ and R⁶ are identical or different, each denoting a hydrogen atom or any of linear or branched alkyl group having 1 to 6 carbon atoms, alkenyl group, and alkoxyl group; and R⁵ denotes any of linear or branched alkyl group having 1 to 6 carbon atoms, alkenyl group, alkoxyl group, and carboxyl group.

The compound represented by the general formula (2) above includes the following: 4,5-dihydroxyindoline, 5,6-dihydroxyindoline, 6,7-dihydroxyindoline, N-methyl-5,6-dihydroxyindoline, N-ethyl-5,6-dihydroxylindoline, N-hexyl-5,6-dihydroxyindoline, 2-methyl-5,6-dihydroxyindoline, 3-methyl-5,6-dihydrxoxyindoline, 4-hydroxyindoline, 2,3-dimethyl-5,6-dihydroxyindoline, 2-methyl-5-ethyl-6-hydroxyindoline, 2-methyl-5-hydroxy-6-β-hydroxyethylindoline, 4-hydroxypropylindoline, 2-hydroxy-3-methoxyindoline, 4-hydroxy-5-methoxyindoline, 6-hydroxy-7-methoxyindoline, 6-hydroxy-5-methoxyindoline, 6-hydroxyindoline, 5-hydroxyindoline, 7-hydroxyindoline, 7-aminoindoline, 5-aminoindoline, 4-aminoindoline, 5,6-dihydroxyindoline carboxylic acid, and 1-methyl-5,6-dihydroxyindoline and a salt thereof.

The above-mentioned dye precursors may be used alone or in combination with one another according to the color desired.

The content of the dye precursor may be properly established according to the frequency of use and the form of composition. Usually, it is 0.01-10%, preferably 0.1-5%, of the total amount of the composition.

The composition may contain, in addition to the above-mentioned ingredients, such optional ingredients as pH buffer, surface active agent, thickener (e.g., hydroxyethylcellulose and xanthan gum), perfume, preservative, UV light absorber, antioxidant, disinfectant, and pearlescent pigment. The surface active agent includes anionic surface active agents (such as α-olefinsulfonate, alkanesulfonate, fatty acid alkyl ether carboxylate, salt of N-acylamino acid, and C₁₂-C₁₈ saturated or unsaturated fatty acid acyl glutamic acid ester), amphoteric surface active agents (such as alkylbetaine, alkylamidebetaine, and hydroxysulfobetaine), cationic surface active agents (such as mono- or dialkyl quaternary ammonium salt), and nonionic surface active agents (such as polyoxyethylene alkyl ether and fatty acid alkylolamide).

The composition may be incorporated with silicone derivatives (such as dimethylpolysiloxane, amino-modified silicone, and polyether-modified silicone) for improvement in hair touch.

The hair dye composition according to the present invention is characterized in that the above-mentioned ingredients are dissolved in carbonated water as a solvent. This carbonated water prevents reactions between the dye precursor and the laccase, with the result that the hair dye composition gives rise to very few aggregates as the reaction product. In other words, the carbonated water contains carbon dioxide gas dissolved therein and this carbon dioxide gas drives away oxygen, thereby preventing the enzyme from reaction with oxygen. Not only does the carbonated water keep the hair dye composition in an atmosphere of carbon dioxide gas during its manufacturing process, but it also keeps the hair dye composition in an atmosphere of carbon dioxide during its storage. Consequently, the carbonated water prevents the dye precursor from forming aggregates and greatly contributes to the storage stability of the product.

The carbonated water used in the present invention is not specifically restricted so long as it is an aqueous solution containing carbon dioxide dissolved therein. Commercial carbonated water may be used. The concentration of carbon dioxide should be 0.1% to saturation.

The composition should have pH 5.0-9.0, preferably pH 6.0-8.0. An excessively high pH causes skin irritation.

The hair dye composition of the present invention is prepared in one-pack type by dissolving, dispersing, or emulsifying the laccase and other ingredients mentioned above in carbonated water. The resulting hair dye composition may take the form of aerosol, cream, gel, liquid, or foam as required.

The hair dye composition according to the present invention is of one-pack type which is convenient for consumers' use, unlike the most conventional permanent hair dye compositions of two-pack type which need the mixing of the first and second packs before use. Owing to carbonated water contained therein, it prevents the dye precursor from forming aggregates during production and storage and hence remains very stable regardless of this form -- aerosol, cream, gel, or liquid.

### EXAMPLE

In what follows, the invention will be described with reference to examples and comparative examples, which are not intended to restrict the scope thereof. (In examples, "%" means "% by weight".)

### Examples 1 to 4 and Comparative Examples 1 to 4

Hair dye compositions in aerosol form were prepared in the usual way according to the formulations shown in Table 1. They were tested for the following items. The results are shown in Table 1.

### Presence of aggregates:

After preparation, compositions were allowed to stand for one month at 25°C and 45°C and then visually examined for the presence or absence of aggregates.
- ⓞ :: none
- ○ :: a few
- Δ :: some
- × :: many

### Hair-dyeing ability (ΔE) :

A wisp of dry goat white hair (about 10 g) is shampooed. After dewatering, the wet hair (17 g) is rapidly treated with a freshly prepared sample of the composition shown in Table 1 (3 g each). The treated hair is allowed to stand at 30°C for a prescribed period of time. Finally, the dyed hair is rinsed, dried, shampooed, and air dried. The thus obtained hair sample is tested for the L value, a value, and b value by using a chroma meter (Model SE2000 made by Nippon Denshokusha) The hair-dyeing ability is rated according to the color difference (ΔE) from undyed hair.
The greater the value of ΔE, the better the hair-dyeing ability.

### Color migration:

A wisp of human black hair (about 10 g) is dyed with a freshly prepared sample of the composition shown in Table 1. The dyed hair is shampooed and dried with a white towel. The towel is visually examined for migrated color.
- ⓞ :: none
- ○ :: slightly noticeable
- Δ :: apparently noticeable
- × :: severely colored

**Table 1**

| Components (%) | | Examples | | | | Comparative Examples | | | |
|---|---|---|---|---|---|---|---|---|---|
| | | 1 | 2 | 3 | 4 | 1 | 2 | 3 | 4 |
| p-Phenylenediamine | | 1.5 | 1.5 | - | 1 | 1.5 | 1.5 | - | 1 |
| p-Aminophenol | | 0.1 | 0.1 | 0.1 | 0.1 | 0.1 | 0.1 | 0.1 | 0.1 |
| m-Aminophenol | | - | - | 0.08 | 0.08 | - | - | 0.08 | 0.08 |
| Toluene-2,5-diamine sulfate | | - | - | 2 | 2 | - | - | 2 | 2 |
| m-Phenylenediamine | | 0.15 | 0.15 | - | - | 0.15 | 0.15 | - | - |
| Resorcinol | | - | - | - | 0.1 | - | - | - | 0.1 |
| Laccase | | 3 | 0.1 | 5 | 10 | 3 | 0.1 | 5 | 0.05 |
| Monoethanolamine | | s.a. | s.a. | s.a. | s.a. | s.a. | s.a. | s.a. | s.a. |
| Carbonated water | | balance | balance | balance | balance | - | - | - | - |
| Purified water | | - | - | - | - | balance | balance | balance | balance |
| LPG (4.0 kg/cm²) | | 4 | 4 | 4 | 4 | 4 | 4 | 4 | 4 |
| Total | | 100.0 | 100.0 | 100.0 | 100.0 | 100.0 | 100.0 | 100.0 | 100.0 |
| pH (adjusted with monoethanolamine) | | 7.0 | 7.0 | 7.0 | 7.0 | 7.0 | 7.0 | 7.0 | 7.0 |
| Presence or absence of aggregates | Immediately after production | ⓞ | ⓞ | ⓞ | ⓞ | Δ | Δ | Δ | Δ |
| | After one month, at 25°C | ⓞ | ⓞ | ⓞ | ⓞ | Δ~× | × | Δ~× | × |
| | After one month, at 45°C | ⓞ | ⓞ | ⓞ | ⓞ | × | × | × | × |
| Hair-dyeing ability (ΔE). immediately after production | | 30 | 35 | 32 | 36 | 26 | 29 | 27 | 29 |
| Color migration, immediately after production | | ⓞ | ⓞ | ⓞ | ⓞ | ○ | ○ | Δ | Δ |

| | | | | | | | | | |
|---|---|---|---|---|---|---|---|---|---|
| s.a.: suitable amount | | | | | | | | | |

### Example 5

A hair dye composition in gel form was prepared in the usual way according to the following formulation. The resulting composition was packed into an aluminum tube. The thus obtained sample was tested in the same way as in Example 1. It exhibited good stability, good hair dyeing ability, and non-color migration.

| | |
|---|---|
| p-Phenylenediamine | 1 % |
| p-Aminophenol | 0.1 |
| m-Aminophenol | 0.2 |
| Laccase | 1 |
| Xanthan gum | 0.5 |
| Hydroxyethylcellulose | 0.5 |
| Carbonated water | Balance |
| Total | 100.0% |
| pH : 7 (adjusted with monoethanolamine) | |

### Examples 6 and 7

A hair dye composition in cream form was prepared in the usual way according to the following formulation. The resulting composition was packed into an aluminum tube. The thus obtained sample was tested in the same way as in Example 1. It exhibited good stability, good hair dyeing ability, and non-color migration.

### Example 6

| | |
|---|---|
| p-Phenylenediamine | 0.5 % |
| Toluene-2,5-diamine sulfate | 1 |
| Hydroxypropyl-β-cyclodextrin | 20 |
| Decaglyceryl monostearate | 3 |
| Cetostearyl alcohol | 0.5 |
| Stearic acid | 0.8 |
| Xanthan gum | 1 |
| Laccase | 1 |
| Carboxymethylcellulose | 1 |
| Sodium hydroxide | suitable amount |
| Carbonated water | Balance |
| Total | 100.0% |
| pH : 7 (adjusted with sodium hydroxide) | |

### Example 7

| | |
|---|---|
| p-Phenylenediamine | 2 % |
| Toluene-2,5-diamine sulfate | 1 |
| m-Phenylenediamine | 0.1 |
| m-Aminophenol | 0.8 |
| Hydroxypropyl-β-cyclodextrin | 45 |
| POE (10) cetyl ether | 8 |
| Stearyl alcohol | 2.5 |
| Oleyl alcohol | 5 |
| Behenyl alcohol | 2 |
| Cetyl alcohol | 2 |
| Stearyltrimethylammonium chloride | 1 |
| Laccase | 0.5 |
| Glycerin | 2 |
| Triethanolamine | suitable amount |
| Carbonated water | Balance |
| Total | 100.0% |
| pH : 7 (adjusted with triethanolamine) | |

### Example 8 (Permanent hair dye composition of one-pack type, in foam form)

| (Stock solution) | |
|---|---|
| p-Phenylenediamine | 1.0 % |
| 2,5-Diaminotoluene sulfate | 2.0 |
| m-Phenylenediamine | 0.5 |
| p-Aminophenol | 0.5 |
| 2-(2'-Hydroxyethylamino)-5-aminotoluene sulfate | 0.15 |
| Oleic acid | 0.2 |
| Oleyl alcohol | 0.2 |
| β-Cyclodextrin | 1.0 |
| Laccase | 1.0 |
| Xanthan gum | 0.5 |
| Acetylcysteine | 0.5 |
| Sorbitan monolaurate | 1.0 |
| Ethanol | 10.0 |
| Glycolic acid | 0.2 |
| Carbonated water | Balance |
| Total | 100.0% |
| pH : 7.5 (adjusted with monoethanolamine) | |

The stock solution prepared as mentioned above was packed into an aerosol can. After clinching in a vacuum, the aerosol can was filled with LPG (as a propellant at 2.0 kg) in such an amount that the ratio of stock solution to propellant is 95 : 5 (by weight). Thus there was obtained the desired hair dye composition in aerosol form.

The LPG as a propellant may be replaced by any of nitrogen, carbon dioxide gas, dinitrogen oxide gas, CFCs 11, 12 and 114. They may be used alone or in combination with one another. The aerosol may be of direct spray type (packed in aluminum cans or tin cans) or of dual-container type (piston type, back-in type, or EXXEL type).

The hair dye composition was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in blue black. This color was the same as that obtained with the fresh composition.

### Example 9 (Permanent hair dye composition of one-pack type, in foam form)

| (Stock solution) | |
|---|---|
| 5,6-Dihydroxyindoline hydrobromide | 1.0 % |
| 5,6-Dihydroxyindole hydrochloride | 1.0 |
| N-Ethyl-5,6-dihydroxyindole hydrochloride | 0.05 |
| Linoleic acid | 0.2 |
| Oleyl alcohol | 0.2 |
| β-cyclodextrin | 1.0 |
| Laccase | 3.0 |
| Hydroxyethylcellulose | 0.5 |
| Acetylcysteine | 1.0 |
| Sodium coconut oil fatty acid acyl-glutamate | 1.0 |
| Ethanol | 10.0 |
| Lactic acid | 0.2 |
| Carbonated water | Balance |
| Total | 100.0% |
| pH : 7.5 (adjusted with monoethanolamine) | |

The stock solution prepared as mentioned above was packed into an aerosol can. After clinching in a vacuum, the aerosol can was filled with LPG (as a propellant at 2.0 kg) in such an amount that the ratio of stock solution to propellant is 95 : 5 (by weight). Thus there was obtained the desired hair dye composition in aerosol form.

The hair dye composition was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in black. This color was the same as that obtained with the fresh composition.

### Example 10 (Permanent hair dye composition of one-pack type, in creamy foam form)

| (Stock solution) | |
|---|---|
| 2,5-Diaminotoluene sulfate | 2.0 % |
| m-Phenylenediamine | 0.5 |
| m-Aminophenol | 0.5 |
| Resorcinol | 0.1 |
| o-Aminocresol | 0.05 |
| Oleic acid | 0.2 |
| β-cyclodextrin | 1.2 |
| Laccase | 5.0 |
| Xanthan gum | 0.05 |
| Stearyltrimethylammonium chloride | 0.2 |
| Cetostearyl alcohol | 0.6 |
| POE (20) hydrogenated castor oil triisostearate | 0.2 |
| Sorbitan monostearate | 0.1 |
| Methyl p-hydroxybenzoate | 0.3 |
| Glycolic acid | 0.2 |
| Carbonated water | Balance |
| Total | 100.0% |
| pH : 7.5 (adjusted with sodium hydroxide) | |

The stock solution prepared as mentioned above was packed into an aerosol can. After clinching in a vacuum, the aerosol can was filled with LPG (as a propellant at 4.0 kg) in such an amount that the ratio of stock solution to propellant is 95 : 5 (by weight). Thus there was obtained the desired creamy hair dye composition in aerosol form.

The hair dye composition was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in dark brown. This color was the same as that obtained with the fresh composition.

### Example 11 (Permanent hair dye composition of one pack type, in creamy foam form)

| (Stock solution) | |
|---|---|
| 5,6-Dihydroxyindoline hydrobromide | 1.0 % |
| 5,6-Dihydroxyindole hydrochloride | 1.0 |
| N-Methyl-5,6-dihydroxyindline hydrobromide | 0.05 |
| Linoleic acid | 0.2 |
| β-Cyclodextrin | 1.0 |
| Laccase | 3.0 |
| Cetostearyltrimethylammonium chloride | 0.2 |
| Cetostearyl alcohol | 0.6 |
| POE (20) hydrogenated castor oil monoisostearate | 0.2 |
| Sorbitan monostearate | 0.1 |
| Methyl p-hydroxybenzoate | 0.3 |
| Lactic acid | 0.2 |
| Carbonated water | Balance |
| Total | 100.0% |
| pH : 7.5 (adjusted with monoethanolamine) | |

The stock solution prepared as mentioned above was packed into an aerosol can. After clinching in a vacuum, the aerosol can was filled with LPG (as a propellant at 4.0 kg) in such an amount that the ratio of stock solution to propellant is 95 : 5 (by weight). Thus there was obtained the desired hair dye composition in aerosol form.

The hair dye composition was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in black. This color was the same as that obtained with the fresh composition.

### Example 12 (Permanent hair dye composition of one-pack type, in cream form)

| | |
|---|---|
| 5,6-Dihydroxyindoline hydrobromide | 1.0 % |
| 5,6-Dihydroxyindole hydrochloride | 1.0 |
| N-Methyl-5,6-dihydroxyindline hydrobromide | 0.05 |
| N-Methyl-5,6-dihydroxyindole hydrochloride | 0.05 |
| 2,5-Diaminotoluene sulfate | 0.01 |
| β-Cyclodextrin | 1.0 |
| Laccase | 10.0 |
| Alkyltrimethylammonium chloride | 0.5 |
| Coconut oil fatty acid acyl-L-arginine ethyl-D,L-pyrrolidone carboxylate | 0.5 |
| Cetostearyl alcohol | 2.0 |
| Oleyl alcohol | 1.0 |
| POE (40) hydrogenated castor oil | 0.75 |
| POE (20) stearyl ether | 0.75 |
| Sorbitan sesquistearate | 1.0 |
| Methyl p-hydroxybenzoate | 0.3 |
| Propylene glycol | 5.0 |
| Glycolic acid | 0.2 |
| Carbonated water | Balance |
| Total | 100.0% |
| pH : 7.5 (adjusted with monoethanolamine) | |

The hair dye composition was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in dark brown. This color was the same as that obtained with the fresh composition.

### Example 13 (Permanent hair dye composition of one-pack type, in cream form)

| | |
|---|---|
| 2,5-Diaminotoluene sulfate | 2.0 % |
| 2,6-Diaminopyridine | 0.05 |
| N,N-Bis(β-hydroxyl)-p-phenylenediamine | 0.1 |
| 2-Amino-5-o-phenol | 0.5 |
| 2-(2'-Hydroxyethylamino)-5-aminotoluene | 0.15 |
| Linoleic acid | 0.2 |
| β-Cyclodextrin | 1.0 |
| Laccase | 1.0 |
| Stearyltrimethylammonium chloride | 0.5 |
| Behenyltrimethylammonium chloride | 0.5 |
| Cetostearyl alcohol | 2.0 |
| Oleyl alcohol | 1.0 |
| POE (40) glycerin triisostearate | 0.75 |
| POE (20) lauryl ether | 0.75 |
| Sorbitan monostearate | 1.0 |
| Methyl p-hydroxybenzoate | 0.3 |
| Propylene glycol | 5.0 |
| Lactic acid | 0.2 |
| Carbonated water | Balance |
| Total | 100.0% |
| pH : 7.5 (adjusted with monoethanolamine) | |

The hair dye composition was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in bright brown. This color was the same as that obtained with the fresh composition.

### Example 14 (Permanent hair dye composition of one-pack type, in treatment form)

| | |
|---|---|
| 2,5-Diaminotoluene sulfate | 5.0 % |
| 2-Amino-4-nitrophenol | 3.0 |
| 5-Amino-o-cresol | 1.0 |
| p-Aminophenol | 1.0 |
| Oleic acid | 0.5 |
| Linoleic acid | 0.5 |
| β-Cyclodextrin | 2.0 |
| Laccase | 10.0 |
| Stearyltrimethylammonium chloride | 0.5 |
| Cetyltrimethylammonium chloride | 0.5 |
| Cetostearyl alcohol | 4.0 |
| Oleyl alcohol | 1.0 |
| Ethyl oleate | 0.5 |
| Isopropyl palmitate | 0.5 |
| Liquid paraffin | 1.0 |
| Bees wax | 0.5 |
| POE (40) hydrogenated castor oil triisostearate | 0.25 |
| POE (20) hydrogenated castor oil triisostearate | 0.25 |
| POE (30) stearyl ether | 0.75 |
| Sorbitan monostearate | 1.0 |
| Glycerin monostearate | 0.5 |
| Methyl p-hydroxybenzoate | 0.3 |
| Propylene glycol | 5.0 |
| Glycolic acid | 0.2 |
| Carbonated water | Balance |
| Total | 100.0% |
| pH : 7.5 (adjusted with sodium hydroxide) | |

The hair dye composition was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in reddish brown. This color was the same as that obtained with the fresh composition. The hair treated with this hair dye composition gave a good touch due to treatment effect.

### Example 15 (Permanent hair dye composition of one-pack type, in treatment form)

| | |
|---|---|
| 5,6-Dihydroxyindoline hydrobromide | 1.0 % |
| 5,6-Dihydroxyindole hydrochloride | 1.0 |
| N-Methyl-5,6-dihydroxyindoline hydrobromide | 0.5 |
| N-Methyl-5,6-dihydroxyindole hydrochloride | 0.5 |
| 5-Aminoindole hydrochloride | 0.25 |
| 2,3-Dimethyl-5,6-dihydroxyindoline hydrobromide | 0.25 |
| Oleic acid | 0.5 |
| Linoleic acid | 0.5 |
| β-Cyclodextrin | 2.0 |
| Laccase | 5.0 |
| Stearyltrimethylammonium chloride | 0.5 |
| Cetyltrimethylammonium chloride | 0.5 |
| Cetostearyl alcohol | 4.0 |
| Oleyl alcohol | 1.0 |
| Ethyl oleate | 0.5 |
| Isopropyl palmitate | 0.5 |
| Liquid paraffin | 1.0 |
| Bees wax | 0.5 |
| POE (40) hydrogenated castor oil triisostearate | 0.25 |
| POE (20) hydrogenated castor oil triisostearate | 0.25 |
| POE (30) stearyl ether | 0.75 |
| Sorbitan monostearate | 1.0 |
| Glycerin monostearate | 0.5 |
| Methyl p-hydroxybenzoate | 0.3 |
| Propylene glycol | 5.0 |
| Lactic acid | 0.2 |
| Carbonated water | Balance |
| Total | 100.0% |
| pH : 7.5 (adjusted with monoethanolamine) | |

The hair dye composition was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in dark gray black. This color was the same as that obtained with the fresh composition. The hair treated with this hair dye composition gave a good touch due to treatment effect.

### Example 16 (Hair dye composition of one-pack type, in gel form)

| | |
|---|---|
| p-Phenylenediamine | 1.0 % |
| 2,5-Diaminotoluene sulfate | 2.0 |
| m-Phenylenediamine | 0.5 |
| p-Aminophenol | 0.5 |
| 2-(2'-Hydroxyethylamino)-5-aminotoluene sulfate | 0.15 |
| Oleic acid | 0.2 |
| β-Cyclodextrin | 1.0 |
| Laccase | 5.0 |
| Xanthan gum | 0.5 |
| Hydroxyethylcellulose | 1.0 |
| POE (40) lauryl ether | 1.0 |
| POE (30) hydrogenated castor oil | 1.0 |
| Ethanol | 10.0 |
| Glycolic acid | 0.2 |
| Carbonated water | Balance |
| Total | 100.0% |
| pH : 7.5 (adjusted with monoethanolamine) | |

The hair dye composition was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in blue black. This color was the same as that obtained with the fresh composition.

### Example 17 (Hair dye composition of one-pack type, in gel form)

| | |
|---|---|
| 5,6-Dihydroxyindoline hydrobromide | 1.0 % |
| 5,6-Dihydroxyindole hydrochloride | 1.0 |
| N-Ethyl-5,6-dihydroxyindole hydrochloride | 0.05 |
| Oleic acid | 0.2 |
| β-Cyclodextrin | 1.0 |
| Laccase | 10.0 |
| Xanthan gum | 0.5 |
| Hydroxyethylcellulose | 1.0 |
| POE (40) lauryl ether | 1.0 |
| POE (30) hydrogenated castor oil | 1.0 |
| Ethanol | 10.0 |
| Glycolic acid | 0.2 |
| Carbonated water | Balance |
| Total | 100.0% |
| pH : 7.5 (adjusted with monoethanolamine) | |

The hair dye composition was stored for six months at room temperature. After storage, an adequate amount of the composition was applied to 1.0 g of white hair. The treated hair was allowed to stand for 20 minutes and then rinsed and shampooed. After drying, the treated hair was found to have been dyed in black. This color was the same as that obtained with the fresh composition.

The hair dye composition of one-pack type according to the present invention has greatly improved storage stability, with laccase reacting very little with the dye precursor to form aggregates, owing to the carbonated water contained therein, and yet it produces a good dyeing effect.

## Claims

1. A laccase-containing oxidation hair dye composition of one-pack type in which carbonated water is used as a solvent.
